# EUROPÄISCHE PATENTSCHRIFT

(11) **EP 1 564 261 B1**
(45) Veröffentlichungstag und Bekanntmachung des Hinweises auf die Patenterteilung: **25.05.2016**
(21) Anmeldenummer: 05002070.0
(22) Anmeldetag: 01.02.2005
(51) Int. Cl.: C09C 1/00, C09D 5/36

(54) **Interferenzpigmente**
Interference pigments
Pigments Interferentiels

(30) Priorität: 09.02.2004 DE 102004006360; 28.10.2004 DE 102004052544
(43) Veröffentlichungstag der Anmeldung: 17.08.2005
(73) Patentinhaber: Merck Patent GmbH, 64293 Darmstadt (DE)
(72) Erfinder: Schmidt, Christoph, Dr., 65830 Kriftel (DE); Delp, Tanja, 64287 Darmstadt (DE); Schoen, Sabine, Dr., 45701 Herten (DE)

(56) Entgegenhaltungen:
- EP-A- 0 753 545
- EP-A- 1 469 041
- WO-A-98/53011
- WO-A-03/006558
- US-A1- 2003 047 115

## Beschreibung

Die vorliegende Erfindung betrifft Interferenzpigmente mit einem intensiven Farbshift auf der Basis von plättchenförmigen, transparenten niedrigbrechenden Substraten und deren Verwendung, insbesondere in Farben, Lacken, Druckfarben, Kunststoffen, als Dotiermittel für die Lasermarkierung von Kunststoffen und Papieren, als Additiv im Lebensmittel- und Pharmabereich und in kosmetischen Formulierungen.

Glanz- oder Effektpigmente werden in vielen Bereichen der Technik eingesetzt, insbesondere im Bereich der Autolacke, der dekorativen Beschichtung, im Kunststoff, in Farben, Druckfarben sowie in kosmetischen Formulierungen.

Glanzpigmente, die einen winkelabhängigen Farbwechsel zwischen mehreren Interferenzfarben zeigen, sind aufgrund ihres Farbenspiels von besonderem Interesse für Autolacke sowie bei fälschungssicheren Wertschriften.

Besondere Bedeutung besitzen dabei Perlglanzpigmente auf mineralischer Basis, wie sie beispielsweise aus der US 2003/047115 A1, EP-A-1 469 041, WO 98/53011 A, EP-A-0 753 545 und WO 03/006558 bekannt sind. Perlglanzpigmente werden durch Beschichtung eines anorganischen, plättchenförmigen Trägers mit einer hochbrechenden, meist oxidischen, Schicht hergestellt. Die Farbe dieser Pigmente wird durch wellenlängenselektive Teilreflektion und Interferenz des reflektierten bzw. transmittierten Lichts an den Medium/Oxid- bzw. Oxid/Substrat-Grenzflächen hervorgerufen.

Die Interferenzfarbe dieser Pigmente wird von der Dicke der Oxidschicht bestimmt, z.B. wird der Farbton eines Grünpigments durch eine im optischen Sinn einzelne hochbrechende Schicht erzeugt, deren optische Dicke ein Reflexionsmaximum im sichtbaren Wellenlängenbereich bei ca. 500 nm hervorruft. Diese Wellenlänge nimmt das menschliche Auge als die Farbe Grün wahr. Im Falle eines Maximums erster Ordnung ist der Intensitätsverlauf jedoch so breit, dass im ganzen Bereich des sichtbaren Lichts so viel Licht reflektiert wird, dass das menschliche Auge einen sehr hellen, aber farblosen Eindruck wahrnimmt.

Gemäß den - insbesondere aus der Vergütung optischer Bauteile - bekannten Regeln der Optik dünner Schichten steigt die Intensität des reflektierten Lichtes bei Anordnung von mehreren Schichten mit abwechselnd hohen und niedrigen Brechzahlen gegenüber einer Einfachschicht stark an. So wird durch Aufbringen eines TiO₂-SiO₂-TiO₂-Schichtsystems auf Glimmerpartikel die Intensität des reflektierten Lichts im Vergleich zu einem TiO₂-Einfachschichtsystem um ca. 60 % angehoben. Demzufolge wird das Profil des durch Interferenz reflektierten Lichts wesentlich ausgeprägter, so dass für ein solches mehrschichtiges System eine intensive und brillante Reflexionsfarbe erwartet werden muss. Pigmente dieses Typs sind in der DE 196 18 569 A1 beschrieben.

Aus dem Stand der Technik sind beispielsweise grüne Interferenzpigmente bekannt auf der Basis von Glimmerplättchen. Glimmerplättchen besitzen in der Regel eine sehr breite Streuung der Schichtdicke und verhalten sich daher in Bezug auf die Interferenzfarbe neutral. Damit stellen Perlglanzpigmente, die eine einzelne hochbrechende Umhüllung von Glimmer aufweisen, optische Einfachschichtsysteme dar, d.h., die Interferenzfarbe wird ausschließlich von der Schichtdicke der hochbrechenden Metalloxidschicht bestimmt. Der coloristische Gestaltungsspielraum eines Glimmer/Metalloxid-Grünpigmentes ist daher sehr eingeschränkt. Zudem besitzen Glimmerteilchen aufgrund ihrer Schichtstruktur Unebenheiten auf der Oberfläche, die Streuung verursachen und damit die Transparenz und die koloristische Qualität des Produktes herabsetzen. Darüber hinaus zeigt Glimmer eine mehr oder weniger stark ausgeprägte graubraune Körperfarbe. Diese Eigenschaft setzt die Transparenz weiter herab und beeinflusst die Absorptionsfarbe der Anwendungsmedien in ungewünschter Weise.

Aufgabe der vorliegenden Erfindung ist es daher ein Interferenpigment, insbesondere ein grünes Interferenzpigment, zur Verfügung zu stellen, das sich insbesondere durch hohe Transparenz, rein weiße Körperfarbe und einen starken Farbflop auszeichnet, die über einen ausschließlichen Farbeffekt, z.B. Grüneffekt, Blaueffekt, etc., hinausgehen.

Überraschenderweise wurde nun gefunden, dass ein reales, mit einer dünnen TiO₂-Schich beschichtetes transparentes niedrigbrechendes Plättchen, wie z. B. ein SiO₂-Plättchen, einen kräftigen, z.B. einen grünen, Farbeindruck hervorruft und gleichzeitig einen außergewöhnlichen und intensiven Farbflop aufweist: Die Farbverschiebung verläuft nicht zwangsläufig wie bei Interferenzfarben üblich von langwelligen zu kurzwelligen Farben, sondern kann auch in der Gegenrichtung, z.B. von dem kurzwelligeren Grün zum langwelligeren Rot verlaufen. Dies gelingt durch eine Beschichtung von dünnen Plättchen, wie z.B. SiO₂-Plättchen, mit einer TiO₂-Schicht, deren Dicke genau auf die Dicke der Plättchen eingestellt wird.

Im Vergleich zu einfach beschichteten Pigmenten, z.B. Grünpigmenten, auf Glimmerbasis zeigen die erfindungsgemäßen Pigmente folgende Eigenschaften:
- ausgezeichnete Transparenz im Anwendungsmedium
- rein weiße Körperfarbe
- kräftige, z.B. grüne, Interferenzfarbe
- sehr hellen Glanz
- starke Farbverschiebung
- bei geeigneter Abstimmung der Schichtdicken von Substrat und Beschichtung ist die Realisierung eines Farbshifts von langwelligen im steilen zu kurzwelligen Farben im flachen Beobachtungswinkel, insbesondere bei grünen Interferenzpigmenten von Grün nach Rot, möglich.

Zusätzlich zu diesen Eigenschaften zeichnen sich die erfindungsgemäßen Pigmente gegenüber den bekannten Interferenzpigmenten durch folgende Merkmale aus:
- kräftiger Glitzer-Effekt
- abstimmbare Gelb-Blau-Tönung der grünen Farbe

Die genannte Farbtönung kann durch Abstimmung der TiO₂-Schichtdicke und durch Wahl der transparenten Plättchen, wie z.B. SiO₂ Plättchen, verschiedener Dicken in weiten Bereichen variiert werden, ohne dass der Eindruck eines Interferenzpigmentes verloren geht.

Gegenstand der Erfindung sind gemäß Anspruch 1 daher hochchromatische, insbesondere grüne, Interferenzpigmente auf der Basis von plättchenförmigen transparenten niedrigbrechenden Substraten, die eine hochbrechende Beschichtung bestehend aus TiO₂ mit einer Schichtdicke von 70 - 160 nm und optional eine äußere Schutzschicht aufweisen.

Gegenstand der Erfindung ist weiterhin die Verwendung der erfindungsgemäßen Interferenzpigmente in Farben, Lacken, Druckfarben, Kunststoffen, Knopfpasten, keramischen Materialien, Gläsern, zur Saatgutbeschichtung, als Additiv zum Laserschweißen von Kunststoffen, als Dotiermittel bei der Lasermarkierung oder beim Laserschweißen von Kunststoffen und Papieren, als Additiv zur Einfärbung im Lebensmittel-und Pharmabereich und insbesondere in kosmetischen Formulierungen. Weiterhin sind die erfindungsgemäßen Pigmente auch zur Herstellung von Pigmentpräparationen sowie zur Herstellung von Trockenpräparaten, wie z. B. Granulaten, Chips, Pellets, Briketts, etc., geeignet. Die Trockenpräparate sind insbesondere für Druckfarben und für kosmetische Formulierungen geeignet.

Geeignete Basissubstrate für die erfindungsgemäßen Interferenzpigmente sind Substrate mit einem Brechungsindex < 1,9, z. B. plättchenförmige SiO₂-Plättchen, wie sie beispielsweise in der WO 93/08237 beschrieben werden. Weiterhin ist neben den genannten SiO₂-Plättchen jedes dem Fachmann bekannte plättchenförmige, transparente Substrat geeignet, wie z. B. Al₂O₃-Plättchen, Glasplättchen, natürlicher oder synthetischer Glimmer, und plättchenförmige Kunststoffpartikel. Ganz besonders bevorzugte Substrate sind SiO₂-Plättchen. Die besonderen Eigenschaften, wie die abstimmbare Farbtönung und insbesondere deren Winkelabhängigkeit, werden durch eine definierte mittlere Dicke mit enger Dickenverteilung maßgeblich hervorgerufen.

Die Standardabweichung der Dicke der Substratplättchen beträgt ≤ 6 % bezogen auf deren mittlere Dicke liegen.

Mittlere Dicke = Summe aller Dickenwerte / Anzahl der Messungen Die Standardabweichung bezieht sich auf die prozentuale Abweichung von 66 % der Einzelmeßwerte von dem berechneten Wert der mittleren Dicke.

Die Größe des Basissubstrats ist an sich nicht kritisch und kann auf den jeweiligen Anwendungszweck abgestimmt werden. In der Regel haben die plättchenförmigen transparenten Substrate eine mittlere Dicke zwischen 0,2 und 0,8 µm. Die Ausdehnung in den beiden anderen Dimensionen beträgt zwischen 5 und 100 µm und ganz besonders bevorzugt bei 5 - 60 µm.

Zur Erzielung eines intensiven Farbeffekts, z.B. eines Grün- oder Blaueffekts, mit überlagerter, winkelabhängiger Farbtönung ist es wichtig, dass die mittlere Dicke der einzelnen Plättchen innerhalb einer Standardabweichung bei ≤ 6 % liegt.

Vorzugsweise beträgt der Formfaktor (aspect ratio: Durchmesser/Dicke-Verhältnis) des Substrats 1 - 1000, insbesondere 3 - 500 und ganz besonders bevorzugt 5 - 200.

Die Dicke der TiO₂-Schicht und des Substrats ist wesentlich für die optischen Eigenschaften des Pigmentes. Die Dicke der Schicht muss genau eingestellt werden und an die mittlere Dicke der Substratplättchen angepasst sein. Die Dicke der TiO₂-Schicht beträgt 70 - 160 nm.

Die erfindungsgemäßen Pigmente lassen sich leicht herstellen durch die Erzeugung einer hochbrechenden TiO₂-Interferenzschicht mit genau definierter Dicke und glatter Oberfläche auf den feinteiligen, plättchenförmigen Substraten. Das TiO₂ liegt in der Rutil-Modifikation vor. Insbesondere bevorzugt sind SiO₂-Plättchen, die mit einer Rutilschicht belegt sind.

Als niedrigbrechende Substrate sind alle anorganische und organischen transparenten Materialen geeignet, die sich in Form feinteiliger Plättchen mit enger Dickenverteilung herstellen lassen und einen Brechungsindex ≤ 1,8 aufweisen. Als organische Substrate kommen unter anderem Polymere, wie z.B. Polyester (z.B. PET), Polycarbonate, Polyimide, Polymethacrylate, in Frage. Besonders bevorzugte anorganische Substrate sind Interferenzpigmente, insbesondere auf Basis von SiO₂, Al₂O₃, plättchenförmigen Einkristallen und Glasplättchen, die auch mit einer dünnen SiO₂-Schicht belegt sein können.

Die Metalloxidschicht wird vorzugsweise nasschemisch aufgebracht, wobei die zur Herstellung von Perlglanzpigmenten entwickelten nass-chemischen Beschichtungsverfahren angewendet werden können. Derartige Verfahren sind z. B. beschrieben in DE 14 67 468, DE 19 59 988, DE 20 09 566, DE 22 14 545, DE 22 15 191, DE 22 44 298, DE 23 13 331, DE 25 22 572, DE 31 37 808, DE 31 37 809, DE 31 51 343, DE 31 51 354, DE 31 51 355, DE 32 11 602, DE 32 35 017 oder auch in weiteren dem Fachmann bekannten Patentdokumenten und sonstigen Publikationen.

Bei der Nassbeschichtung werden die Substratpartikel in Wasser suspendiert und mit ein oder mehreren hydrolysierbaren Titansalzen bei einem für die Hydrolyse geeigneten pH-Wert versetzt, der so gewählt wird, dass die Metalloxide bzw. Metalloxidhydrate direkt auf den Plättchen ausgefällt werden, ohne dass es zu wesentlichen Nebenfällungen kommt. Der pH-Wert wird üblicherweise durch gleichzeitiges Zudosieren einer Base und/oder Säure konstant gehalten. Anschließend werden die Pigmente abgetrennt, gewaschen und bei 50-150 °C getrocknet und gegebenenfalls 0,1-3 h geglüht, wobei die Glühtemperatur im Hinblick auf die jeweils vorliegende Beschichtung optimiert werden kann. In der Regel liegen die Glühtemperaturen zwischen 250 und 1000 °C, vorzugsweise zwischen 350 und 950 °C.

Weiterhin kann die Beschichtung auch in einem Wirbelbettreaktor durch Gasphasenbeschichtung erfolgen, wobei z. B. die in EP 0 045 851 A1 und EP 0 106 235 A1 zur Herstellung von Perlglanzpigmenten vorgeschlagenen Verfahren entsprechend angewendet werden können.

Der Farbton der Pigmente kann unter Erhaltung des Interferenzeffekts, z.B. Grüninterferenzeffekts, in sehr weiten Grenzen durch unterschiedliche Wahl der Belegungsmengen bzw. der daraus resultierenden Schichtdicken variiert werden. Der winkelabhängige Farbshift von z.B. Grün in steilen Beobachtungswinkeln zu Rot in flachen Beobachtungswinkeln bleibt dabei weitgehend erhalten. Die Farbtonvarianz betrifft dabei insbesondere den Gelb- bzw. Blauanteil. Die Feinabstimmung für einen bestimmten Farbton kann über die reine Mengenwahl hinaus durch visuell oder messtechnisch kontrolliertes Anfahren der gewünschten Farbe erreicht werden.

Zur Erhöhung der Licht-, Wasser- und Wetterstabilität empfiehlt es sich häufig, in Abhängigkeit vom Einsatzgebiet, das fertige Pigment einer Nachbeschichtung oder Nachbehandlung zu unterziehen. Als Nachbeschichtungen bzw. Nachbehandlungen kommen beispielsweise die in den DE-PS 22 15 191, DE-OS 31 51 354, DE-OS 32 35 017 oder DE-OS 33 34 598 beschriebenen Verfahren in Frage. Durch diese Nachbeschichtung wird die chemische Stabilität weiter erhöht oder die Handhabung des Pigments, insbesondere die Einarbeitung in unterschiedliche Medien, erleichtert. Zur Verbesserung der Benetzbarkeit, Dispergierbarkeit und/oder Verträglichkeit mit den Anwendungsmedien können funktionelle Beschichtungen aus Al₂O₃ oder ZrO₂ oder deren Gemische bzw. Mischphasen auf die Pigmentoberfläche aufgebracht werden. Weiterhin sind organische, bzw. organisch/anorganisch kombinierte Nachbeschichtungen möglich, z.B. mit Silanen, wie beispielsweise beschrieben in der EP 0090259, EP 0 634 459, WO 99/57204, WO 96/32446, WO 99/57204, U.S. 5,759,255, U.S. 5,571,851, WO 01/92425 oder in J.J. Ponjee, Philips Technical Review, Vol. 44, No. 3, 81 ff. und P.H. Harding J.C. Berg, J. Adhesion Sci. Technol. Vol. 11 No. 4, S. 471-493.

Die erfindungsgemäßen Interferenzpigmente sind einfach und leicht zu handhaben. Die Pigmente können durch einfaches Einrühren in das Anwendungssystem eingearbeitet werden. Ein aufwendiges Mahlen und Dispergieren der Pigmente ist nicht erforderlich.

Da die erfindungsgemäßen Interferenzpigmente starken Glanz mit hoher Transparenz und rein weißer Körperfarbe verbinden, lassen sich mit ihnen besonders wirksame Effekte in den verschiedenen Anwendungsmedien erzielen ohne die Absorptionsfarbe wesentlich zu beeinflussen.

Es versteht sich von selbst, dass für die verschiedenen Anwendungszwecke die Interferenzpigmente auch vorteilhaft in Abmischung mit organischen Farbstoffen, organischen Pigmenten oder anderen Pigmenten, wie z. B. transparenten und deckenden Weiß-, Bunt- und Schwarzpigmenten sowie mit plättchenförmigen Eisenoxiden, organischen Pigmenten, holographischen Pigmenten, LCPs (Liquid Crystal Polymers), und herkömmlichen transparenten, bunten und schwarzen Glanzpigmenten auf der Basis von metalloxidbeschichteten Glimmer- und SiO₂-Plättchen, etc., verwendet werden können. Die Interferenzpigmente können in jedem Verhältnis mit handelsüblichen Pigmenten und Füllstoffen gemischt werden.

In den verschiedenen Anwendungen kann das erfindungsgemäße Pigment auch mit weiteren Farbmitteln jeden Typs, z. B. organischen und/oder anorganischen Absorptionspigmenten und Farbstoffen, mehrschichtigen Interferenzpigmenten, wie z.B. Timiron®, Sicopearl® (BASF AG), ChromaFlair® (Flex Products Inc.), BiOCl-Pigmenten, Fischsilber, Metallpigmente z. B. von der Fa. Eckart, kombiniert werden. Dabei sind den Mischungsverhältnissen und Konzentration keine Grenzen gesetzt.

Die erfindungsgemäßen Pigmente sind mit einer Vielzahl von Farbsystemen kompatibel, vorzugsweise aus dem Bereich der Lacke, Farben und Druckfarben. Für die Herstellung der Druckfarben für z. B. den Tiefdruck, Flexodruck, Offsetdruck, Offsetüberdrucklackierung, ist eine Vielzahl von Bindern, insbesondere wasserlösliche Typen, geeignet, wie sie z. B. von den Firmen BASF, Marabu, Pröll, Sericol, Hartmann, Gebr. Schmidt, Sicpa, Aarberg, Siegberg, GSB-Wahl, Follmann, Ruco oder Coates Screen INKS GmbH vertrieben werden. Die Druckfarben können auf Wasserbasis oder Lösemittelbasis aufgebaut sein. Weiterhin sind die Pigmente auch für die Lasermarkierung von Papier und Kunststoffen, sowie für Anwendungen im Agrarbereich, z. B. für Gewächshausfolien, sowie z. B. für die Farbgebung von Zeltplanen, geeignet.

Das erfindungsgemäße Interferenzpigment kann zur Pigmentierung von Lacken, Druckfarben, Kunststoffen, Agrarfolien, Saatgutbeschichtung, Lebensmitteleinfärbungen, Knopfpasten, Arzneimittelüberzügen oder kosmetischen Formulierungen, wie Lippenstifte, Nagellacke, Presspuder, Shampoos, Seifen, lose Puder und Gele, verwendet werden. Die Konzentration der Pigmentmischung im zu pigmentierenden Anwendungssystem liegt in der Regel zwischen 0,1 und 70 Gew.%, vorzugsweise zwischen 0,1 und 50 Gew.% und insbesondere zwischen 0,5 und 10 Gew.%, bezogen auf den Gesamtfestkörpergehalt des Systems. Sie ist in der Regel abhängig vom konkreten Anwendungsfall.
In Kunststoffen enthaltend das erfindungsgemäße grüne Interferenzpigment, vorzugsweise in Mengen von 0,01 bis 50 Gew.%, insbesondere 0,1 bis 7 Gew.%, lassen sich besonders ausgeprägte Sparkle-Effekte erzielen.

Im Lackbereich, insbesondere im Automobillack, wird das Interferenzpigment, auch für 3-Schichtaufbauten in Mengen von 0,1-20 Gew.%, vorzugsweise 1 bis 10 Gew.%, eingesetzt.

Im Lack hat das erfindungsgemäße Interferenzpigment den Vorteil, dass der angestrebte Glanz durch eine einschichtige Lackierung (Einschichtsystem bzw. Base coat im 2-Schichtaufbau) erzielt wird. Im Vergleich mit Lackierungen, die beispielsweise ein Mehrschichtpigment auf Basis von Glimmer bzw. ein herkömmliches, auf einem Substrat mit breiter Dickenverteilung basierendes Perlglanzpigment, statt des erfindungsgemäßen Pigments enthalten, zeigen Lackierungen mit dem erfindungsgemäßen Pigment eine deutlichere Tiefenwirkung und einen stärker ausgeprägten Glanzeffekt.

Das erfindungsgemäße Interferenzpigment kann auch vorteilhaft in der dekorativen und pflegenden Kosmetik eingesetzt werden. Die Einsatzkonzentration reicht von 0,01 Gew.% im Shampoo bis zu 100 Gew. % bei losen Pudern. Bei einer Mischung der Interferenzpigmente mit sphärischen Füllstoffen, z. B. SiO₂, kann die Konzentration bei 0,01-70 Gew.% in der Formulierung liegen. Die kosmetischen Produkte, wie z.B. Nagellacke, Presspuder, Shampoos, lose Puder und Gele, zeichnen sich durch besonders interessante Farbeffekte und einen intensiven Farbshift aus. Der Farbflöp-Effekt in Nagellack kann gegenüber herkömmlichen Nagellacken mit Hilfe der erfindungsgemäßen Pigmente deutlich gesteigert werden.

Weiterhin kann das erfindungsgemäße Pigment in Badezusätzen, Zahnpasten und zur Veredlung von Lebensmitteln, z. B. Masse-Einfärbung und/oder Überzüge von Bonbons, Weingummi, wie z.B. Gummibärchen, Pralinen, Lakritze, Konfekt, Zuckerstangen, Puddings, Brausegetränke, Limonaden, etc., oder als Überzug, z.B. bei Dragees und Tabletten im Pharmabereich, eingesetzt werden.

Das erfindungsgemäße Pigment kann weiterhin mit handelsüblichen Füllern gemischt werden. Als Füllstoffe sind z.B. zu nennen natürlicher und synthetischer Glimmer, Nylon Powder, reine oder gefüllte Melaminharze, Talcum, Gläser, Kaolin, Oxide oder Hydroxide von Aluminium, Magnesium, Calcium, Zink, BiOCl, Bariumsulfat, Calciumsulfat, Calciumcarbonat, Magnesiumcarbonat, Kohlenstoff, sowie physikalische oder chemische Kombinationen dieser Stoffe. Bezüglich der Partikelform des Füllstoffes gibt es keine Einschränkungen. Sie kann den Anforderungen gemäß z. B. plättchenförmig, sphärisch oder nadelförmig sein.

Selbstverständlich können die erfindungsgemäßen Interferenzpigmente in den Formulierungen auch mit jeder Art von kosmetischen Roh- und Hilfsstoffen kombiniert werden. Dazu gehören u.a. Öle, Fette, Wachse, Filmbildner, Konservierungsmittel und allgemein anwendungstechnische Eigenschaften bestimmende Hilfsstoffe, wie z. B. Verdicker und rheologische Zusatzstoffe, wie z.B. Bentonite, Hektorite, Siliciumdioxide, Ca-Silicate, Gelatinen, hochmolekulare Kohlenhydrate und/oder oberflächenaktive Hilfsmittel etc.

Die die erfindungsgemäßen Interferenzpigmente enthaltenden Formulierungen können dem lipophilen, hydrophilen oder hydrophoben Typ angehören. Bei heterogenen Formulierungen mit diskreten wässrigen und nichtwässrigen Phasen können die erfindungsgemäßen Pigmente in jeweils nur einer der beiden Phasen enthalten oder auch über beide Phasen verteilt sein.

Die pH-Werte der Formulierungen können zwischen 1 und 14, bevorzugt zwischen 2 und 11 und besonders bevorzugt zwischen 5 und 8 liegen.

Den Konzentrationen der erfindungsgemäßen Pigmente in der Formulierung sind keine Grenzen gesetzt. Sie können - je nach Anwendungsfall - zwischen 0,001 (rinse-off-Produkte, z.B. Duschgele) und 100 % (z.B. Glanzeffekt-Artikel für besondere Anwendungen) liegen.
Die erfindungsgemäßen Pigmente können weiterhin auch mit kosmetischen Wirkstoffen kombiniert werden. Geeignete Wirkstoffe sind z.B. Insect Repellents, UV A/BC-Schutzfilter (z.B. OMC, B3, MBC), Anti-Ageing-Wirkstoffe, Vitamine und deren Derivate (z.B. Vitamin A, C, E etc.), Selbstbräuner (z.B. DHA, Erytrolyse, u.a.) sowie weitere kosmetische Wirkstoffe wie z.B. Bisabolol, LPO, Ectoin, Emblica, Allantoin, Bioflavanoide und deren Derivate.

Bei der Pigmentierung von Bindemittelsystemen z. B. für Farben und Druckfarben für den Tiefdruck, Offsetdruck oder Siebdruck, oder als Vorprodukt für Druckfarben, hat sich der Einsatz der erfindungsgemäßen Interferenzpigmente in Form von hochpigmentierten Pasten, Granulaten, Pellets, etc., als besonders geeignet erwiesen. Das Pigment wird in der Regel in die Druckfarbe in Mengen von 2-35 Gew.%, vorzugsweise 5-25 Gew.%, und insbesondere 8-20 Gew.% eingearbeitet. Offsetdruckfarben können die Pigmente bis zu 40 Gew.% und mehr enthalten. Die Vorprodukte für die Druckfarben, z.B. in Granulatform, als Pellets, Briketts, etc., enthalten neben dem Bindemittel und Additiven bis zu 98 Gew.% des erfindungsgemäßen Pigments. Die Druckfarben enthaltend das erfindungsgemäße Pigment zeigen reinere Farbtöne, insbesondere Grünfarbtöne, als mit herkömmlichen Effektpigmenten. Die Partikeldicken der erfindungsgemäßen Interferenzpigmente sind relativ gering und bedingen daher eine besonders gute Verdruckbarkeit.

Die erfindungsgemäßen Interferenzpigmente sind weiterhin geeignet zur Herstellung von fließfähigen Pigmentpräparationen und Trockenpräparaten, insbesondere für Druckfarben, enthaltend ein oder mehrere erfindungsgemäße Pigmente, Bindemittel und optional ein oder mehrere Additive.

Gegenstand der Erfindung sind somit auch Formulierungen enthaltend das erfindungsgemäße Interferenzpigment.

Gegenstand der Erfindung sind insbesondere Formulierungen, die neben dem erfindungsgemäßen Interferenzpigment mindestens einen Bestandteil ausgewählt aus Absorptionsmitteln, Adstringenzien, antimikrobiellen Stoffen, Antioxidantien, Antiperspirantien, Antischaummitteln, Antischuppenwirkstoffen, Antistatika, Bindemitteln, biologischen Zusatzstoffen, Bleichmitteln, Chelatbildnern, Desodorierungsmitteln, Emollentien, Emulgatoren, Emulsionsstabilisatoren, Farbstoffen, Feuchthaltemitteln, Filmbildnern, Geruchsstoffen, Geschmacksstoffen, Insect Repellents, Konservierungsmitteln, Korrosionsschutzmitteln, kosmetischen Ölen, Lösungsmitteln, Oxidationsmitteln, pflanzlichen Bestandteilen, Puffersubstanzen, Reduktionsmitteln, Tensiden, Treibgasen, Trübungsmitteln, UV-Filtern und UV-Absorbern, Vergällungsmitteln, Viskositätsreglern, Parfüm und Vitaminen enthalten.

Die nachfolgenden Beispiele sollen die Erfindung erläutern, ohne sie jedoch zu begrenzen.

### Beispiele

### Beispiel 1: Interferenzpigment mit Colortravel von intensiv Grün nach Rot

100 g SiO₂-Plättchen (Teilchengröße 5-50 µm, mittlere Dicke 450 nm, Standardabweichung der Dicke: ca. 5 %) werden in 2I VE-Wasser suspendiert und unter starkem Rühren auf 80 °C erhitzt. Zu dieser Mischung wird bei pH 1,6 eine Lösung von 12 g SnCl₄ x 5 H₂O und 40 ml Salzsäure (37 %) in 360 ml VE-Wasser zudosiert. Anschließend wird bei einem pH-Wert von 1,6 eine Menge von 460 ml TiCl₄-Lösung (400 g TiCl₄/l) zudosiert. Der pH-Wert wird bei der Zugabe der SnCl₄ x 5 H₂O-Lösung und TiCl₄-Lösungen jeweils mit NaOH-Lösung (32 %) konstant gehalten. Anschließend wird der pH-Wert mit Natronlauge (32 %) auf 5,0 eingestellt und 15 Minuten nachgerührt. Zur Aufarbeitung wird das Pigment abfiltriert, mit 20 I VE-Wasser gewaschen, bei 110 °C getrocknet und 30 min. bei 850 °C geglüht. Man erhält ein Interferenzpigment mit intensiver brillantgrüner Farbe, starkem Glanz und hoher Transparenz. Beim Übergang zu flachen Beobachtungswinkeln zeigt das Pigment eine rote Interferenzfarbe.

### Anwendungsbeispiele

### Beispiel A: Duschgel

**Phase A**

| **Rohstoff** | **Bezugsquelle** | **INCI** | **[%]** |
|---|---|---|---|
| Pigment aus Beispiel 1 | Merck KGaA | | 0,10 |
| Keltrol T | Kelco | Xanthan Gum | 0,75 |
| Wasser, demineralisiert | | Aqua (Water) | 64,95 |

**Phase B**

| **Rohstoff** | **Bezugsquelle** | **INCI** | **[%]** |
|---|---|---|---|
| Plantacare 2000 UP | Coqnis GmbH | Decyl Glucoside | 20,00 |
| Texapon ASV 50 | Cognis GmbH | Sodium Laureth Sulfate, Sodium Laureth-8 Sulfate, Magnesium Laureth Sulfate, Magnesium Laureth-8 Sulfate, Sodium Oleth Sulfate, Magnesium Oleth Sulfate | 3,60 |
| Bronidox L | Cognis GmbH | Propylene Glycol, 5-Bromo-5-Nitro-1,3-Dioxane | 0,20 |
| Parfümöl Everest 79658 SB (gestrichen) | Haarmann & Reimer GmbH | Parfum | 0,05 |
| 1 % FD&C Blue No. 1 in Wasser | BASF AG | Aqua (Water), Cl 42090 (FD&C Blue No. 1) | 0,20 |

**Phase C**

| **Rohstoff** | **Bezugsquelle** | **INCI** | **[%]** |
|---|---|---|---|
| Citronensäure Monohydrat | Merck KGaA/Rona® | Citric Acid | 0,15 |
| Wasser, demineralisiert | | Aqua (Water) | 10,00 |

### Herstellung:

Für Phase A das Interferenzpigment in das Wasser einrühren. Keltril T unter Rühren langsam einstreuen und rühren bis es gelöst ist. Die Phasen B und C nacheinander hinzufügen und dabei langsam rühren bis alles homogen verteilt ist. pH-Wert auf 6,0 bis 6,4 einstellen.

### Beispiel B: - Nagellack

| **Rohstoff** | **Bezugsquelle** | **INCI** | **[%]** |
|---|---|---|---|
| Pigment aus Beispiel 1 | Merck KGaA | | 2,00 |
| Thixotrope Nagellack-Base 1348 | International Lacquers S.A. | Toluene, Ethyl Acetate, Butyl Acetate, Nitrocellulose, Tosylamide/Formaldehyde Resin, Dibutyl Phthalate, Isopropyl Alcohol, Stearalkonium Hectorite, Camphor, Acrylates Copolymer, Benzophenone-1 | 98,00 |

### Herstellung:

Das Interferenzpigment wird zusammen mit der Lackbase eingewogen, gut mit einem Spatel von Hand vermischt und anschließend 10 min bei 1000 Upm gerührt.

### Beispiel C: - Lacksystem

| | |
|---|---|
| 90 Gew. % | Hydroglasur BG/S farblos (Wasserlack der Fa. Ernst Diegel GmbH) |
| 10 Gew. % | grünes Interferenzpigment aus Beispiel 1 |

Lackieren durch Aufsprühen bei 80 °C
5 min vortrocknen bei 80 °C
20 min einbrennen bei 180 °C

### Beispiel D : - Kunststoff

1 kg Polystyrolgranulat werden in einem Taumelmischer mit 5 g Haftmittel gleichmäßig benetzt. Dazu werden dann 42 g grünes Interferenzpigment aus Beispiel 1 zugegeben und 2 min lang gemischt. Dieses Granulat wird auf einer Spritzgießmaschine unter üblichen Bedingungen zu Stufenplättchen mit den Maßen 4 x 3 x 0,5 cm verarbeitet. Die Stufenplättchen zeichnen sich durch ihren ausgeprägten Sparkle-Effekt aus.

### Beispiel E: - Einfärbung von Süßwaren

Rohware: Brause-Bonbons weiß

Sprühlösung:
94 % alkoholische Schellacklösung der Fa. Kaul
6 % grünes Interferenzpigment aus Beispiel 1

Die Brausebonbons werden mit einer Interferenzpigment/Schellacklösung besprüht bis der gewünschte Farbauftrag erreicht ist. Eine nachträgliche Trocknung mit Kaltluft ist möglich.

## Patentansprüche

1. Interferenzpigmente auf der Basis von niedrigbrechenden transparenten plättchenförmigen Substraten mit einem Brechungsindex < 1,9, **dadurch gekennzeichnet, dass** die Standardabweichung der Dicke der Substratplättchen ≤ 6 % ist bezogen auf deren mittlere Dicke, wobei die mittlere Dicke zwischen 0,2 und 0,8 µm beträgt und die Ausdehnung in den anderen Dimensionen zwischen 5 und 100 µm beträgt und sie eine hochbrechende Beschichtung bestehend aus TiO₂ in der Rutilmodifikation mit einer Schichtdicke von 70 - 160 nm auf den plättchenförmigen Substraten und optional eine äußere Schutzschicht aufweisen.

2. Interferenzpigmente nach Anspruch 1, **dadurch gekennzeichnet, dass** das transparente plättchenförmige Substrat ein SiO₂-Plättchen, Al₂O₃-Plättchen, natürliches oder synthetisches Glimmerplättchen oder ein Glasplättchen ist.

3. Interferenzpigmente nach Anspruch 1 oder 2, **dadurch gekennzeichnet, dass** das plättchenförmige Substrat ein SiO₂-Plättchen ist.

4. Verfahren zur Herstellung der Interferenzpigmente nach einem oder mehreren der Ansprüche 1 bis 3, **dadurch gekennzeichnet, dass** die Beschichtung der Substrate nasschemisch durch hydrolytische Zersetzung von Metallsalzen in wässrigem Medium oder durch thermische Zersetzung nach dem CVD- oder PVD-Verfahren erfolgt.

5. Verwendung der Interferenzpigmente nach einem oder mehreren der Ansprüche 1 bis 3 in Farben, Knopfpasten, Lacken, Druckfarben, Sicherheitsdruckfarben, Kunststoffen, keramischen Materialien, Gläsern, zur Saatgutbeschichtung, als Dotiermittel für die Lasermarkierung von Kunststoffen und Papieren, als Additiv zum Laserschweißen von Kunststoffen, als Additiv zur Farbgebung im Lebensmittel- und Pharmabereich, in kosmetischen Formulierungen und zur Herstellung von Pigmentpräparationen und Trockenpräparaten.

6. Pigmentpräparationen enthaltend ein oder mehrere Bindemittel, optional ein oder mehrere Additive und ein oder mehrere Interferenzpigmente nach einem oder mehreren der Ansprüche 1 bis 3.

7. Trockenpräparationen wie Pellets, Granulate, Chips, Briketts enthaltend Interferenzpigmente nach einem oder mehreren der Ansprüche 1 bis 3.

## Claims

1. Interference pigments based on low-refractive-index, transparent, flake-form substrates having a refractive index < 1.9, **characterised in that** the standard deviation of the thickness of the substrate flakes is ≤ 6%, based on their average thickness, where the average thickness is between 0.2 and 0.8 µm and the size in the other dimensions is between 5 and 100 µm and they have a high-refractive-index coating consisting of TiO₂ in the rutile modification having a layer thickness of 70 - 160 nm on the flake-form substrates and optionally an outer protective layer.

2. Interference pigments according to Claim 1, **characterised in that** the transparent, flake-form substrate is an SiO₂ flake, Al₂O₃ flake, natural or synthetic mica flake or a glass flake.

3. Interference pigments according to Claim 1 or 2, **characterised in that** the flake-form substrate is an SiO₂ flake.

4. Process for the preparation of the interference pigments according to one or more of Claims 1 to 3, **characterised in that** the coating of the substrates is carried out by wet-chemical methods by hydrolytic decomposition of metal salts in aqueous medium or by thermal decomposition by the CVD or PVD process.

5. Use of the interference pigments according to one or more of Claims 1 to 3 in paints, button pastes, coatings, printing inks, security printing inks, plastics, ceramic materials, glasses, for coating seed, as dopant for the laser marking of plastics and papers, as additive for the laser welding of plastics, as additive for colouring in the foods and pharmaceuticals sectors, in cosmetic formulations and for the preparation of pigment preparations and dry preparations.

6. Pigment preparations comprising one or more binders, optionally one or more additives and one or more interference pigments according to one or more of Claims 1 to 3.

7. Dry preparations, such as pellets, granules, chips or briquettes, comprising interference pigments according to one or more of Claims 1 to 3.

## Revendications

1. Pigments d'interférence basés sur des substrats transparents sous forme de flocons à indice de réfraction faible présentant un indice de réfraction < 1,9, **caractérisés en ce que** l'écart type de l'épaisseur des flocons de substrat est ≤ 6%, sur la base de leur épaisseur moyenne, où l'épaisseur moyenne est entre 0,2 et 0,8 µm et la taille dans les autres dimensions est entre 5 et 100 µm et ils comportent un revêtement à indice de réfraction élevé constitué par TiO₂ selon la modification rutile présentant une épaisseur de couche de 70 - 160 nm sur les substrats sous forme de flocons et en option, une couche de protection externe.

2. Pigments d'interférence selon la revendication 1, **caractérisés en ce que** le substrat transparent sous forme de flocons est un flocon de SiO₂, un flocon d'Al₂O₃, un flocon de mica naturel ou synthétique ou un flocon de verre.

3. Pigments d'interférence selon la revendication 1 ou 2, **caractérisés en ce que** le substrat sous forme de flocons est un flocon de SiO₂.

4. Procédé pour la préparation des pigments d'interférence selon une ou plusieurs des revendications 1 à 3, **caractérisé en ce que** le revêtement des substrats est effectué au moyen de procédés chimiques par voie humide par décomposition hydrolytique de sels de métaux dans un milieu aqueux ou par décomposition thermique au moyen du procédé CVD (dépôt chimique en phase vapeur) ou PVD (dépôt physique en phase vapeur).

5. Utilisation des pigments d'interférence selon une ou plusieurs des revendications 1 à 3 dans des peintures, des pâtes à boutons, des revêtements, des encres d'impression, des encres d'impression de sécurité, des matières plastiques, des matériaux de céramique, des verres, pour former un revêtement sur des semences, en tant que dopant pour le marquage laser de matières plastiques et de papiers, en tant qu'additif pour le soudage laser de matières plastiques, en tant qu'additif pour la coloration dans les secteurs alimentaire et pharmaceutique, dans les formulations cosmétiques et pour la préparation de préparations de pigments et de préparations à sec.

6. Préparations de pigments comprenant un ou plusieurs liant(s), en option un ou plusieurs additif(s) et un ou plusieurs pigment(s) d'interférence selon une ou plusieurs des revendications 1 à 3.

7. Préparations à sec, telles que les pellets, les granules, les chips ou les briquettes, comprenant des pigments d'interférence selon une ou plusieurs des revendications 1 à 3.
